Europäisches Patentamt

European Patent Office

Office européen des brevets

(19)

(11) Publication number: **0 293 130**
**A2**

## (12) EUROPEAN PATENT APPLICATION

(21) Application number: 88304488.5

(22) Date of filing: 18.05.88

(51) Int. Cl.4: **C07K 7/10** , **A61K 37/64** ,
**G01N 33/68**

(30) Priority: 26.05.87 US 54460

(43) Date of publication of application:
30.11.88 Bulletin 88/48

(84) Designated Contracting States:
CH DE FR GB IT LI NL

(71) Applicant: MERCK & CO. INC.
126, East Lincoln Avenue P.O. Box 2000
Rahway New Jersey 07065-0900(US)

(72) Inventor: Rosenblatt, Michael
209 Glenn Road
Ardmore Pennsylvania PA 19003(US)
Inventor: Caporale, Lynn H.
221 Susquehanna Street
Lansdale Pennsylvania PA 19466(US)
Inventor: Nutt, Ruth F.
Hill Road
Green Lane Pennsylvania PA 18054(US)
Inventor: Levy, Jay J.
107 Dalton Road
Paoli Pennsylvania 19301(US)

(74) Representative: Hesketh, Alan, Dr.
European Patent Department Merck & Co.,
Inc. Terlings Park Eastwick Road
Harlow Essex, CM20 2QR(GB)

(54) Dimers of parathyroid hormone antagonists.

(57) The present invention relates to dimers of peptide hormone analogues and their use as inhibitors of their respective naturally occurring peptide hormone. The structure of the dimers is exemplified by $([Tyr^{34}Cys^{39}]PTH(21-39)NH_2)_2$, alpha, epsilon (-$[Tyr^{34}]PTH(21-38))_2LysNH_2$ and (-$[Nle^{8,18},Tyr^{34},Cys^{39}]bPTH(7-39)NH_2)_2$.

## DIMERS OF PARATHYROID HORMONE ANTAGONISTS

### BACKGROUND OF THE INVENTION

This invention relates to the use of peptide hormone analogues for inhibiting the naturally occurring hormone peptide in vivo and in vitro. These peptide hormone analogues when administered to a vertebrate, such as mammals, block the endrocrine activity of the peptide hormone or other analogous molecules. These peptide hormone analogues are also useful in vitro in combination with a bioassay for the naturally occurring hormone. The peptide hormone analogues are useful in treating various diseases caused by hormone excess and in treating hormone dependent tumors. One example of this invention relates to the synthesis of dimers of parathyroid hormone analogues useful for inhibiting the action of parathyroid hormone both in vivo and in vitro.

Analysis of the relation of structure to hormonal function has provided important insights into the mechanism of action of peptide hormones. Each type of peptide hormone has an affinity for specific receptors to which it binds. Upon binding, the peptide hormone acts either directly or causes a change in the intracellular concentration of a second messenger molecule such as cyclic AMP, cyclic GMP, or calcium ions. These second messenger molecules, in turn, cause changes in the metabolism or physiology of the cell. These changes in cell metabolism or physiology are directly or indirectly dependent upon the binding of the peptide hormone to its specific cell surface receptor. Therefore, if the cell surface receptor is blocked then the hormone effect is also blocked.

Peptide hormone analogues have long been known as a method through which the biochemistry of hormones can be studied and evaluated. Endocrinologists have long desired a method for producing a class of peptide hormone analogues which would allow the blocking of specific hormone receptors without activating a change in the second messenger molecules, thereby avoiding the hormone induced metabolic changes.

Rosenblatt et al., U.S. Patent 4,423,037 and the publications referred to therein describe the structure of certain peptide hormone analogues and their binding to cell receptors. In particular, these publications describe the properties of parathyroid hormone analogues and their physiological properties.

Scientific efforts over a period of many years have sought to understand the interaction between peptide hormones and the cell surface receptor specific for each peptide hormone. One of the peptide hormones, parathyroid hormone, has been studied by using analogues of parathyroid hormone (PTH). One objective of these studies has been to understand the binding of the peptide hormone to the cell surface receptor such that an analogue could be constructed which would bind with the same or greater affinity than the naturally occurring hormone. This analogue would enable the peptide hormone analogue of parathyroid hormone to be used to block the effect of the naturally occurring parathyroid hormone. One of the major problems encountered in this search for a clinically and pharmacologically effective parathyroid hormone analogue was the problem of agonist activity. Agonist activity is the property of the peptide hormone analogue to itself stimulate the change in second messengers which brings about the physiological change associated with the naturally occurring hormone. Therefore, the problem was to create hormone analogues which would bind with high affinity to the appropriate hormone cell surface receptor but not stimulate a change in the second messenger concentration, that is, not act as hormone itself. These analogues could then be used in treating hormone related diseases.

It is an object of the present invention to provide dimers of PTH antagonists. These dimers greatly decrease the macroscopic dissociation off-rate for a multimeric receptor. It is theorized that one arm of the dimer is not free to move away from the receptor as long as the other arm is bound and thus the dimer is more likely to reassociate. Further, once one arm is bound to the receptor, the second is more likely to bind, thus increasing the on-rate.

Another object of the present invention is to provide novel PTH dimers. Another object of the present invention is to provide a novel method of inhibiting the action of PTH through the administration of novel PTH dimers. Still another object of the invention is to provide PTH dimers wherein amino acid modifications result in binding to all the surface receptor without activating the second messenger molecule. The above and other objects are accomplished by the present invention in the manner more fully described below.

### SUMMARY OF THE INVENTION

The present invention provides a peptide which comprises a dimer of PTH antagonist. The dimer can be linked by a bridge at positions 35 to 39. Cystine or lysine are particularly useful bridges at these positions. A [Cys[39]] or [Lys[39]] bridge is par-

ticularly effective. The dimer can have arms containing from 19 to 37 amino acids, particularly from 19 to 33 amino acids. The particular PTH dimers have one of the following structures:
([Tyr$^{34}$,Cys$^{39}$]PTH(21-39)NH$_2$)$_2$;    ([Nle$^{8,18}$, Tyr$^{34}$,Cys$^{39}$]bPTH(7-39)NH$_2$)  α∈([Tyr$^{34}$]PTH (21-38))$_2$LysNH$_2$α∈([Nle$^{8,18}$,Tyr$^{34}$]hPTH (7-38))$_2$LysNH$_2$. The PTH can be human parathyroid hormone (hPTH), bovine parathyroid hormone (bPTH) or rat parathyroid hormone (rPTH).

The present invention also provides a method of inhibiting the action of parathyroid hormone comprising the administration of therapeutically effective amount of a parathyroid hormone dimer described above. The present invention also provides a method of treating osteoporosis or hypercalcemia comprising the administration of a therapeutically effective amount of a parathyroid hormone dimer described above. A method of treating hyperparathyroidism comprising the administration of a therapeutically effective amount of the parathyroid hormone dimers of this invention is also provided. A method of treating hyperparathyroidism expressed as a hypercalcemic crisis, renal failure or hypertension is also provided. A method of treating the disease state produced by a tumor or other cell overproducing a peptide hormone-like molecule and method of treating immune diseases wherein the disease state comprises inflammation, an allergic response, or hyperactive lymphocytes is also provided by the novel peptide hormone dimers of the present invention.

## DETAILED DESCRIPTION OF THE INVENTION

Various other objects, features and attendant advantages of the present invention will be more fully appreciated as the same becomes better understood from the following detailed description.

Extensive structure and activity studies have now led to the design of peptide hormone dimers which have high binding affinity for their respective cell surface receptors while not stimulating the production of second messenger molecules. An example of such a peptide hormone dimer is (-[Tyr$^{34}$,Cys$^{39}$]       PTH(21-39)NH$_2$)$_2$;       (-[Nle$^{8,18}$,Tyr$^{34}$,Cys$^{39}$]bPTH  (7-39)NH$_2$); α,∈([Tyr$^{34}$]-PTH(21-38))$_2$LysNH$_2$; α∈ ([Nle$^{8,18}$,Tyr$^{34}$]hPTH(7-38)-)$_2$LysNH$_2$ which inhibits PTH in vivo but does not act as an agonist.

Agonist activity is dependent upon the presence of the N-terminal amino acid sequence. The removal of the two to six end terminal amino acids results in the loss of most if not all agonist activities. Therefore, the second messenger molecules are not affected by those dimers which have the altered amino terminus. PTH dimers with two to six amino acids removed from the N-terminus produces an inhibitor which still binds with high affinity to the peptide hormone receptor without causing a change in cyclic AMP concentration.

The following is the 34-amino acid sequence of bovine parathyroid hormone (bPTH):
H2N-ALA-VAL-SER-GLU-ILE-GLN-PHE-MET-HIS-ASN-LEU-GLY-LYS-HIS-LEU(15)-SER-SER-MET-GLU-ARG-VAL-GLU-TRP-LEU-ARG-LYS-LYS-LEU-GLN-ASP(30)-VAL-HIS-ASN-PHE-COOH.

The following is the 34-amino acid sequence of human parathyroid hormone (hPTH):
H2N-SER-VAL-SER-GLU-ILE-GLN-LEU-MET-HIS-ASN(10)-LEU-GLY-LYS-HIS-LEU-ASN-SER-MET-GLU-ARG(20)-VAL-GLU-TRP-LEU-ARG-LYS-LYS-LEU-GLN-ASP(30)-VAL-HIS-ASN-PHE-COOH.

The following is the 34-amino acid sequence of rat parathyroid hormone (rPTH):
H2N-ALA-VAL-SER-GLU-ILE-GLN-LEU-MET-HIS-ASN(10)-LEU-GLY-LYS-HIS-LEU-ALA-SER-VAL-GLU-ARG(20)-MET-GLN-TRP-LEU-ARG-LYS-LYS-LEU-GLN-ASP(30)-VAL-HIS-ASN-PHE-COOH.

Fragments of peptide hormones containing the region specific for binding to the cell surface receptor can be used as inhibitors or blocking agents. For parathyroid hormone, the N-terminal 34 amino acids are sufficient to define binding specificity to the parathyroid hormone cell surface receptor. This receptor specificity is further defined by the following publication herein incorporated by reference: M. Rosenblatt, et al., Endocrinology, 107:2, 545-550, 1980 and S. R. Nussbaum, et al., Journal of Biological Chemistry, 255:10183, 1980.

The presence of D-amino acids in peptide hormone in place of L-amino acids results in a peptide resistant to catabolism. However, not all such substitutions result in an active peptide hormone. The insertion of D-tyrosine at position 34 in PTH results in a significant increase in the biological activity of the hormone in addition to increasing stability of the peptide. The utilization of D-amino acids in peptide hormone synthesis is described in the following publications herein incorporated by reference: Coltrera, et al., Bio chemistry, 19:4380-4385, 1980; Rosenblatt et al., Biochemistry, 20:7246-7250, 1981.

The balance of the description will be divided into two sections. Section I will describe the preparation and structure of inhibitors of peptide hormones, Section II will discuss the use of the peptide hormone inhibitors.

## I. Preparation and Structure of Peptide Hormone Inhibitors

The technique of solid-phase peptide synthesis, developed by Merrifield ("Solid-Phase Peptide Synthesis", Advances in Enzymology, 32:221-296, 1969) has been successfully employed in the synthesis of peptide hormones including parathyroid hormone. This method is based on the strategy of having the carboxyl terminus of the peptide linked covalently to a solid support. The desired peptide sequence is prepared by stepwise coupling of single amino acids to a peptide chain growing from the carboxyl toward the amino terminus. Because each amino acid is coupled by nearly the same series of reactions, the need for elaborate strategies in the synthesis is minimized. Solublility is not a major issue during synthesis, because the peptide is linked to a solid support. This method is rapid and it can be utilized by a single worker. It is very convenient for the synthesis of multiple analogues with amino-terminal substitutions, because a single synthesis can be branched in multiple directions near the amino terminus, thereby creating many analogues varying only in the amino terminal region.

## II. Use of Peptide Hormone Inhibitors

The method of inhibiting the action of peptide hormones comprises the administration of a therapeutically effective amount of any peptide hormone or analogue wherein the two N-terminal amino acids are removed and zero or more of the next four N-terminal amino acids are removed sequentially from the N-terminus. These hormone analogues retain specificity for the cell surface receptor without stimulating a physiological response. This method of use applies to the entire peptide hormone or its analogue, or to a fragment of the peptide hormone containing the receptor binding site.

The use of peptide hormone analogues is exemplified by parathyroid hormone analogues. The parathyroid hormone may be of bovine, human, rat, or any vertebrate origin. The analogue may contain all the amino acids except for the modified N-terminal region or it might comprise the N-terminal 7-34 amino acids. Individual amino acids can be substituted to improve stability as exemplified by tyrosine or norleucine in the present invention.

The peptide hormone analogues of this invention can be used in vitro to measure the concentration of naturally occurring peptide hormone. This bioassay procedure is illustrated by a bioassay for parathyroid hormone. The unknown concentration of parathyroid hormone in a solution can be determined by measuring the amount of parathyroid hormone analogue required to inhibit its binding to the parathyroid hormone cell surface receptor. The concentration of PTH analogue required to block the action of parathyroid hormone is a direct indicator of the parathyroid hormone concentration.

Parathyroid hormone analogues can be used to diagnose the etiology of or to treat osteoporosis or hypercalcemia through the administration of a therapeutically effective amount of the parathyroid hormone analogues of this invention. Similarly, other aspects of hyperparathyroidism, such as a hypercalcemic crisis, renal failure or hypertension can be treated through the administration of the parathyroid hormone analogues of this invention.

Tumors and other aberrant cell growth often produce hormone like substances causing a disease state. The use of peptide hormone analogues to block stimulation caused by such hormone like substances can result in the alleviation of the disease state. Therefore, the peptide hormone analogues of the present invention can be administered to treat diseases caused by aberrant production of hormone like substances.

Immune diseases such as inflammation, allergic responses and hyperactive lympocytes can be treated through the administration of peptide hormone analogues which block the action of peptide hormones, such as PTH analogues inhibiting the binding of PTH to cells of the immune system.

The peptide hormone analogues of this invention exhibit both oral and parenteral activity and can be formulated in dosage forms for oral parenteral, rectal, intra-nasal, or topical administration. Solid dosage forms for oral administration include capsules, tablets, pills, powders and granules. In such solid dosage forms, the active compound is admixed with at least one inert diluent such as sucrose, lactose or starch. Such dosage forms can also comprise, as is normal practice, additional substances other than inert diluent. In the case of capsules, tablets, and pills, the dosage forms may also comprise buffering agents. Tablets and pills can additionally be prepared with an enteric coating.

Liquid dosage forms for oral administration include pharmaceutically acceptable emulsion, solutions, suspensions, syrups and elixers containing inert diluents commonly used in the pharmaceutical art. Besides inert diluents, such compositions can also include adjuvants, such as wetting agents, emulsifying and suspending agents, and sweetening. Preparations according to this invention for parenteral administration include sterile aqueous or non-aqueous solutions, suspensions or emulsions. Examples of non-aqueous solvents or vehicles are propylene glycol, polyethylene glycol, vegetable oils such as olive oil and injectable organic esters such as ethyloleate.

Compositions for rectal administration are suppositories which may contain in addition to the active substance,, expients such as cocoa butter or a suppository wax. The dosage of active ingredient in the compositions of this invention may be varied; however it is necessary the amount of the active ingredient shall be such that a suitable dosage form is obtained. The selected dosage form depends upon the desired therapeutic effect, on the route on the administration, and on the duration of the treatment.

Obviously, numerous modifications and variations of the present invention are possible in light of the above teachings. It is therefore to be understood that within the scope of the appended claims, the invention may be practiced otherwise than as specifically described herein.

EXAMPLE 1

## Preparation Dimeric of Peptide Hormone Inhibitors Containing Cystine Bridge

The monomeric peptide intermediate is synthesized by the solid phase method using Cys-(Acm) at the point of cystine bridge formation. the monomeric peptide such as [Tyr$^{34}$Cys(Acm)$^{39}$] PTH(21-39) is obtained from peptide-resin by "low-high" HF cleavage (Tam,JACS 105, 6442-6455 (1983) and purified on Sephedex G-50F eluting with 50% HOAc. Further purification is carried out by HPLC. The monomeric intermediate is converted to the dimeric product such as ([Tyr$^{34}$Cys$^{39}$-[PTH (21-39))$_2$ using I$_2$ in 80% HOAc. The dimeric product is isolated by gel filtration (G-50F, 50%HOAc) and preparative reverse phase HPLC.

EXAMPLE 2

## Preparation of Dimeric Inhibitors Containing a Lysine Bridge

The dimeric peptide such as $\gamma$ ,$\epsilon$-([Tyr$^{34}$] PTH-(21-38))$_2$Lys-NH$_2$ is assembled by the solid phase method by coupling$\alpha$-Fmoc-Lys(Boc) to 4-methylbenzhydryl amine-resin (USB) using standard coupling protocol. The two blocking groups are removed sequentially by first treating the amino acid-resin with piperidine CH$_2$Cl$_2$ followed by standard TFA deblocking of the Boc group. The two free amino groups of Lys are acylated simultaneously with protected amino acids until the desired peptide-resin such as $\epsilon$ ,$\alpha$([Tyr$^{34}$]PTH(21-38))$_2$-Lys-MBHR is prepared. The dimeric product is cleaved from the solid support by "low-high" HF cleavage (Tam, JACS 105: 6447-6455 (1983)) and purified by gel filtration (G-50F,50%HOAc) and preparative reverse phase HPLC.

EXAMPLE 3

## PTH Binding Assay Results

In the adenylate cyclase assay of Caporale, L. et al,Peptides: Structure and Function, Proc. 9th Am. Pept. Symp. p. 663 (1985) the dimer (-[Tyr$^{34}$,Cys$^{39}$]PTH(21-39)NH$_2$)$_2$ exhibited a 25-fold increase in antagonist potency (IC$_{50}$ 20uM) over its monomeric derivative.

## Claims

1. A peptide which comprises a dimer of a PTH antagonist.

2. A peptide according to Claim 1 wherein the arms of the dimer are linked by a bridge at position 35 to 39.

3. A peptide according to Claim 2 wherein the PTH is hPTH, bPTH or rPTH.

4. A peptide according to Claim 3 wherein the arms of the dimer contain from 19 to 37 amino acids.

5. A peptide according to Claim 4 wherein the bridge is cystine.

6. A peptide according to Claim 4 wherein the bridge is lysine.

7. A peptide according to Claim 5 which is (-[Tyr$^{34}$,Cys$^{39}$]PTH(21-39)NH$_2$)$_2$; ([Nle$^{8,18}$, Tyr$^{34}$,Cys$^{39}$]bPTH(7-39)NH$_2$)$_2$.

8. A peptide according to Claim 6 which is alpha, epsilon ([Tyr$^{34}$]PTH(21-38))$_2$LysNH$_2$; alpha, epsilon ([Nle$^{8,18}$,Tyr$^{34}$ ]hPTH(7-38))$_2$ LysNH$_2$.

9. The use of a peptide as claimed in any one of claims 1 to 8 for the preparation of a medicament useful for increasing the affinity of a PTH antagonist for a PTH receptor.

10. An in vitro bioassay of parathyroid hormone, wherein a measured amount of the peptide of Claim I inhibits binding a parathyroid hormone to a PTH receptor in vitro.

11. The use as claimed in claim 9, wherein said medicament is for the treatment of hypercalcemia.

12. The use as claimed in Claim 9, wherein said medicament is for the diagnosing or treating of hyperparathyroidism.

13. The use as claimed in Claim 9, wherein a tumor produces a parathyroid hormone-like substance. 14. The use as claimed in Claim 9, wherein said medicament is for the treatment of an immune disease.

15. The use as claimed in Claim 9, wherein said medicament is for the treatment of hypertension.

16. The use as claimed in Claim 9, wherein said medicament is for the treatment of osteoporosis.

17. A pharmaceutical composition which comprises an effective amount of a peptide of Claim I and a pharmaceutically acceptable carrier.